# EUROPEAN PATENT SPECIFICATION

(11) **EP 1 764 605 B1**
(45) Date of publication and mention of the grant of the patent: **22.09.2021**
(21) Application number: 06076430.5
(22) Date of filing: 17.07.2006
(51) Int. Cl.: G01N 3/42, G01N 33/24, E02D 1/02, G01N 3/40

(54) **Cone penetrometer**
Kegelpenetrometer
Pénétromètre conique

(30) Priority: 22.07.2005 NL 1029595
(43) Date of publication of application: 21.03.2007
(73) Proprietor: VERENIGDE BEDRIJVEN VAN DEN BERG HEERENVEEN HOLDING B.V., NL-8445 PK Heerenveen (NL)
(72) Inventor: Veenstra, Ruurd, 8431 TW Oosterwolde fr (NL)
(74) Representative: EP&C

(56) References cited:
- WO-A2-2005/003728
- US-A- 4 382 384
- US-A- 4 398 414
- US-A- 4 554 819
- US-A1- 2004 118 199
- None

## Description

The invention relates to a cone penetrometer. Cone penetrometers are used with a soil probing device, as is known for example from NL 1012468. The cone penetrometer has a conical nose at the front side, and at the rear side there are coupling means for coupling the penetrometer to a probing rod. The conical nose of the cone penetrometer is pressed into the ground by means of the probing rod, with the soil resistance being measured by measuring means that are present in the penetrometer.

The invention relates more particularly to a cone penetrometer in accordance with the preamble of claim 1.

A cone penetrometer of this type is known.

EP 0 010 988 has shown a cone penetrometer in which a sleeve-shaped load cell is arranged around a main body and a friction sleeve is arranged around the main body and the load cell. The load cell has an internal screwthreaded portion in the vicinity of a rear end, in order to be screwed on to an external screwthreaded portion on a main body. Furthermore, in the vicinity of a front end, load cell has an external screwthreaded portion which is screwed to an internal screwthreaded portion of the friction sleeve. Assembly of the various components is complex, due to the various screwthreaded connections which have to be screwed together.

US 4 398 414 shows an electrical friction sleeve cone penetrometer, which comprises a main body, a load cell, and a friction sleeve. The load cell extends around a center part of the main body. The friction sleeve extends around the entire load cell as well as around front and rear parts of the main body. The load cell is fixedly connected to the main body by means of a secured/locked screw thread connection. With this a rear edge of the load cell lies against a radially outwardly projecting shoulder of the main body.

The load cell is also fixedly connected to the friction sleeve. With this a front edge of a shoulder of the load cell lies against a radially inwardly projecting shoulder of the friction sleeve.

Other examples of measuring devices where a friction sleeve and a load cell are fixedly attached to each other and to a main body, are known from for example US 2004/0118199, WO 2005/003728 and US 4,554,819.

It is an object of the invention to provide an improved cone penetrometer.

According to the invention, this object is achieved by a cone penetrometer that according to the preamble of claim 1 comprises a substantially tubular main body having a front end and a rear end, a conical nose which is coupled to the front end of the main body, and a cylindrical friction sleeve arranged around part of the main body, in such a manner that the main body and the friction sleeve delimit an annular clearance, in which a substantially sleeve-shaped force measurement element is accommodated concentrically with the main body and the friction sleeve, in which the main body comprises a shoulder with a stop face facing towards the front end, in which the friction sleeve comprises a shoulder with a stop face facing towards the rear end, in which the force measurement element has two end portions and a middle portion extending between them, and in which the end portions of the force measurement element have end faces, characterized in that the force measurement element, in the axial direction, is retained only by the stop face which is directed towards the rear and arranged on the friction sleeve and by the stop face which is directed towards the front and arranged on the main body, wherein the stop faces of the main body respectively of the friction sleeve bear against the end faces of the force measurement element, such that the force measurement element is fixed without screw connections between the force measurement element and the friction sleeve and between the force measurement element and the main body.

In the cone penetrometer according to the invention, the force measurement element is fixed without screw connections between the friction sleeve and the main body, thereby simplifying assembly.

Preferred embodiments of the cone penetrometer according to the invention are given in the dependent claims.

The invention also relates to a method for assembling a cone penetrometer according to claim 12. In this method, a substantially sleeve-shaped force measurement element is moved axially over a substantially tubular main body from the front end. On the main body there is arranged a stop face which is directed towards the front end and on to which the force measurement element is pushed.

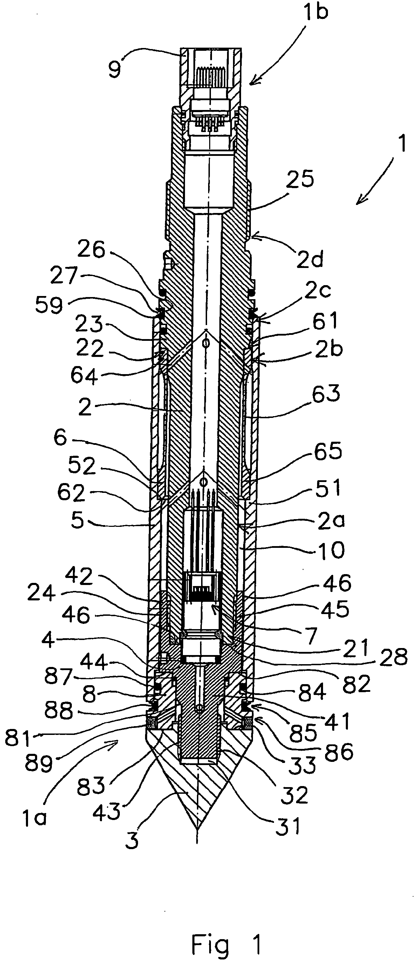

The invention will be explained in more detail with reference to the drawing, in which Fig. 1 shows a preferred embodiment of a cone penetrometer according to the invention.

Fig. 1 shows a cone penetrometer, which is denoted overall by reference numeral 1. The cone penetrometer 1 has a front end 1a and a rear end 1b.

The cone penetrometer 1 has a main body 2 which is substantially tubular and is produced from a single piece. In the axial direction, the main body has roughly four sections of different external diameters. A first section 2a extends from a front end 21 towards the rear. A second section 2b adjoins the first section 2a and has a larger diameter than the first section 2a. A third section or collar section 2c adjoins the second section 2b and has a larger diameter than the second section 2b. A shoulder 23 with a stop face 22 facing towards the front end is formed between the second section 2b and the collar section 2c. In the collar section 2c is arranged at least one circumferential groove 26 with a sealing ring 27.

The collar section 2c is followed by a fourth section or coupling section 2d, which is provided, inter alia, with an external screw thread section 25 for coupling to a probing rod (not shown).

An external screwthreaded portion 24 is arranged on the main body 2 in the vicinity of the front end for coupling to a coupling piece 4, as will be described in more detail further on in the description.

The cone penetrometer 1 also comprises a friction sleeve 5 which is substantially cylindrical in form and is arranged concentrically around the first section 2a, the second section 2b and part of the collar portion 2c of the main body 2. The front end of the friction sleeve 5 extends beyond the front end of the main body 2. At the level of the first and second sections 2a, 2b of the main body 2, the internal diameter of the friction sleeve 5 is larger than the external diameter of the main body 2, so that an annular clearance 10 is formed between the friction sleeve 5 and the main body 2. At the level of the third section 2c, the inner side of the friction sleeve 5 bears against the outer side of the main body 2. A shoulder 51 with a stop face 52 directed towards the rear is formed on the inside approximately in the axial centre of the friction sleeve 5.

A substantially sleeve-shaped, round force measurement element 6 produced as a single piece is accommodated in the clearance 10. The force measurement element 6 has two end portions 64 and 65 and a middle portion 63 extending between them with a smaller wall thickness than the end portions 64, 65. The surface on the inner side of the force measurement element 6 is substantially smooth. The surface of the end portions 64, 65 and the middle portion 63 is also smooth on the outer side. The end portions 64 and 65 have end faces 61 and 62, respectively, against which the stop faces 22 and 52 bear, with the result that the force measurement element 6 is retained in a positively locking manner between the two stop faces 22 and 52. The middle portion 63 is designed to be able to deform when a compressive force is applied to the force measurement element 6 by the friction sleeve 5 via the stop face 52 in the direction of the stop face 22. Strain gauges (not shown) are arranged, in a manner known per se, in a plurality of locations on the outer surface of the middle portion 63, by means of which strain gauges the deformation of the associated portion can be measured. This deformation represents the compressive force exerted on the force measurement element 6 and therefore the frictional force which is exerted on the friction sleeve by the soil when the penetrometer 1 is pressed into the ground.

At the front end 1a of the penetrometer 1 is arranged a nose assembly, which here comprises a conical nose 3, a centring ring 8 and a coupling piece 4.

The conical nose 3 is provided with a blind hole 31 which extends from the rear side 33 towards the front and in which is arranged internal screw thread 32.

The coupling piece 4 has a coupling pin 41 and a substantially sleeve-shaped coupling socket 42, connected to it at one end of the coupling pin 41, the central axis of which coupling socket 42 extends coaxially with the central axis of the coupling pin 41. The coupling socket 42 has a larger external diameter than the coupling pin 41, with the result that a stop face 44 directed towards the front is formed at the transition between them.

The coupling socket 42 has a preferably substantially smooth outer surface 46. Screw thread 45 is arranged at the inner side of the coupling socket 42. A radially inwardly extending annular stop face 46, which faces the insertion side of the socket 42, is formed on the inner side.

External screw thread 43 is arranged on the coupling pin 41 and is used to screw the coupling pin 41 into the hole 31 in the conical nose 3.

The centring ring 8 is located between the coupling socket 42 and the conical nose 3 and has a central hole 81, through which the coupling pin 41 extends. By means of the screw connection between the conical nose 3 and coupling pin 41 of the coupling piece 4, the stop face 44 of the coupling piece 4 is clamped against an end face 82 of the centring ring 8 and the rear side 33 of the conical nose 3 is clamped against the opposite end face 83 of the centring ring 8.

On the outer side, the centring ring 8 is provided with a number of, in the exemplary embodiment three, circumferential grooves 84 to 86, in which are arranged sealing rings 87 to 89. The middle sealing ring 88 for example can be a ring with sealing lips.

In the vicinity of the front end 21 of the main body 2, force measuring means 7 are arranged on the inner side. In the assembled state, the coupling socket 42 is screw-fitted by way of the screw thread 45 on to the external screw thread 24 of the main body 2, specifically so far that the end face 28 of the main body 2 comes to bear against the internal stop face 46 in the coupling socket 42. As a result of the penetrometer 1 being pressed into the ground with the nose 3 at the front, the conical nose 3 is subject to a resistance which is transmitted via the nose assembly, more particularly via the centring ring 8 and the coupling piece 4 at the end face 28 to the main body 2. This force leads to deformation of the front section 21 of the main body 2, which deformation can be measured by means of the force measuring means 7 and is convertable into a measuring signal. The measuring signal represents the force to which the conical nose 3 is subjected during the movement through the soil.

During assembly of the cone penetrometer 1, first of all the force measurement element 6 is arranged around the main body from the front side 21 until the end face 61 bears against the stop face 22.

Then, the coupling piece 4 is screw-fitted on to the front end of the main body 2.

Then, the friction sleeve 5 is pushed from the front side 21 over the main body 2, the coupling piece 4 and over the force measurement element 6 until the stop face 52 bears against the end face 62. At that moment, the rear end face 59 of the friction sleeve 5 contacts the sealing ring 27.

Then, the centring ring 8 is pushed over the coupling pin 41 and partially into the friction sleeve 5, with the front end face coming into contact with the sealing ring 88. Finally, the conical nose 3 is screw-fitted on to the coupling pin 41, resulting in a positive lock of the elements on the main body 2.

As an alternative, it is also possible for the components of the nose assembly to be assembled first of all and then for the nose assembly in its entirety to be screw-fitted to the main body 2 after the force measurement element 6 and the friction sleeve 5 have been pushed around the main body 2.

The use of the coupling piece 4 has the advantage that the conical nose 3 can be used for various types of cone penetrometers. For the conical nose 3 to be arranged on a main body other than the type shown, it merely therefore has to be coupled to a coupling piece which fits to the associated main body. The conical nose is expensive to produce compared to a coupling piece. According to the preferred embodiment it is possible to suffice with just one type of conical nose, for this reason the various cone penetrometers can be produced at lower costs.

Furthermore, it is also possible for the force measurement element 6 to be pushed into the friction sleeve 5 first of all and then for these two components together to be pushed over the main body 2.

A connector 9 with a connector housing and connectors is fitted to the rear end 1b of the cone penetrometer 1 by means of a screw-fit to the main body 2. The connector 9 is connected to the force pick-up means 6 and 7. Data transmission lines can be coupled to the connectors in order to transport the measurement data, for example through the probing rod, to the surface, for further processing by data processing means, for example a computer.

Therefore, the invention provides a cone penetrometer in which fewer screw threads have to be arranged on the main body compared to the cone penetrometer which is known from EP 0 010 988, and no screw threads whatsoever have to be arranged on the friction sleeve and the force measurement element. This makes the penetrometer according to the invention less laborious and cheaper to produce than the known embodiment.

## Claims

1. Cone penetrometer comprising:
- a substantially tubular main body (2) having a front end and a rear end,
- a conical nose (3) which is coupled to the front end of the main body (2), and
- a cylindrical friction sleeve (5) arranged around part of the main body (2), in such a manner that the main body (2) and the friction sleeve (5) delimit an annular clearance (10),
in which a substantially sleeve-shaped force measurement element (6) is accommodated concentrically with the main body (2) and the friction sleeve (5),
in which the main body (2) comprises a shoulder (23) with a stop face (22) facing towards the front end,
in which the friction sleeve (5) comprises a shoulder (51) with a stop face (52) facing towards the rear end,
in which the force measurement element (6) has two end portions (64, 65) and a middle portion (63) extending between them, and
in which the end portions (64, 65) of the force measurement element (6) have end faces (61, 62),
**characterized in that**
the force measurement element (6), in the axial direction, is retained only by the stop face (52) which is directed towards the rear and arranged on the friction sleeve (5) and by the stop face (22) which is directed towards the front and arranged on the main body (2),
wherein the stop faces (22, 52) of the main body (2) respectively of the friction sleeve (5) bear against the end faces (61, 62) of the force measurement element (6), such that the force measurement element (6) is fixed without screw connections between the force measurement element (6) and the friction sleeve (5) and between the force measurement element (6) and the main body (2).

2. Cone penetrometer according to claim 1, in which the conical nose (3) is coupled to the main body (2) by means of a separate coupling piece (4).

3. Cone penetrometer according to claim 2, in which the coupling piece (4) comprises a coupling pin (41), and the conical nose (3) comprises a hole (31) for forming a pin/hole connection, preferably a screw-fit.

4. Cone penetrometer according to claim 3, in which the coupling piece (4) also comprises a coupling socket (42), fixedly connected to the coupling pin (41), for insertion of the front end of the main body (2).

5. Cone penetrometer according to claim 4, in which the coupling socket (42) is provided with an internal screw thread (45) and the end of the main body (2) is provided with an external screw thread (24) for the purpose of forming a screw-fit.

6. Cone penetrometer according to one of the preceding claims, in which the front end of the friction sleeve (5) extends beyond the front end of the main body (2).

7. Cone penetrometer according to one of the preceding claims, in which the main body (2) is produced out of a single piece.

8. Cone penetrometer according to one of the preceding claims, in which an external screw thread (25) is arranged at the rear end of the main body (2) for forming a connection to one end of a probing rod.

9. Cone penetrometer according to one of the preceding claims, in which force measuring means are arranged in the main body (2) for measuring the cone resistance.

10. Cone penetrometer according to one of the preceding claims, in which the force measurement element (6) is formed as a hollow cylinder with a substantially smooth inner side.

11. Cone penetrometer according to one of the preceding claims, in which the force measurement element (6) is formed as a hollow cylinder with a substantially smooth outer side.

12. Method for producing a cone penetrometer comprising a substantially tubular main body (2) having a front end and a rear end, has a shoulder (23) with a stop face (22) facing towards the front end,
in which a substantially sleeve-shaped force measurement element (6), that has two end portions (64, 65) with end faces (61, 62) and a middle portion (63) extending between them, is arranged around part of the main body (2), concentrically with the main body (2),
in which a cylindrical friction sleeve (5), that has a shoulder (51) with a stop face (52) facing towards the rear end, is arranged around a portion of the main body (2) and around the force measurement element (6), so that the stop face (52) of the shoulder (51) thereof which is directed towards the rear bears against the end portion (64) of the force measurement element (6), and
in which the force measurement element (6) is moved over the main body (2) in the axial direction from the front end until it contacts the stop face (22) of the shoulder (23) which is directed towards the front and arranged on the main body (2),
**characterized in that**
first of all the force measurement element (6) is pushed over the main body (2) until it reaches the stop face (22) thereof, and
then the friction sleeve (5) is pushed over the main body (2) and the force measurement element (6) from the front side,
after which a nose assembly comprising a conical nose (3) is coupled to the front of the main body (2),
wherein the stop faces (22, 52) of the main body (2) respectively of the friction sleeve (5) bear against the end faces (61, 62) of the force measurement element (6), so that the force measurement element (6) and the friction sleeve (5) are axially fixed on the main body (2) in a positively locking manner, without screw connections between the force measurement element (6) and the friction sleeve (5) and between the force measurement element (6) and the main body (2).

## Patentansprüche

1. Konuspenetrometer, umfassend:
- einen im Wesentlichen rohrförmigen Hauptkörper (2) mit einem vorderen Ende und einem hinteren Ende,
- eine konische Nase (3), die mit dem vorderen Ende des Hauptkörpers (2) gekoppelt ist, und
- eine zylindrische Reibhülse (5), die um einen Teil des Hauptkörpers (2) auf solche Weise herum angeordnet ist, dass der Hauptkörper (2) und die Reibhülse (5) einen ringförmigen Zwischenraum (10) begrenzen,
wobei ein im Wesentlichen hülsenförmiges Kraftmesselement (6) konzentrisch zum Hauptkörper (2) und zur Reibhülse (5) aufgenommen ist,
wobei der Hauptkörper (2) einen Absatz (23) mit einer Anschlagfläche (22) umfasst, die zu dem vorderen Ende ausgerichtet ist,
wobei die Reibhülse (5) einen Absatz (51) mit einer Anschlagfläche (52) umfasst, die zu dem hinteren Ende ausgerichtet ist,
wobei das Kraftmesselement (6) zwei Endabschnitte (64, 65) aufweist sowie einen mittleren Abschnitt (63), der sich dazwischen erstreckt, und
wobei die Endabschnitte (64, 65) des Kraftmesselements (6) Endflächen (61, 62) aufweisen,
**dadurch gekennzeichnet, dass**
das Kraftmesselement (6) in der axialen Richtung nur durch die Anschlagfläche (52), die zu dem hinteren Ende ausgerichtet und auf der Reibhülse (5) angeordnet ist, und durch die Anschlagfläche (22), die zu dem vorderen Ende ausgerichtet und auf dem Hauptkörper (2) angeordnet ist, gehalten wird,
wobei die Anschlagflächen (22, 52) des Hauptkörpers (2) der jeweiligen Reibhülsen (5) an den Endflächen (61, 62) des Kraftmesselements (6) anliegen, so dass das Kraftmesselement (6) ohne Schraubverbindungen zwischen dem Kraftmesselement (6) und der Reibhülse (5) und zwischen dem Kraftmesselement (6) und dem Hauptkörper (2) fixiert wird.

2. Konuspenetrometer nach Anspruch 1, wobei die konische Nase (3) an dem Hauptkörper (2) mithilfe eines separaten Kopplungsstücks (4) gekoppelt ist.

3. Konuspenetrometer nach Anspruch 2, wobei das Kopplungsstück (4) einen Kopplungsstift (41) umfasst, und die konische Nase (3) eine Bohrung (31) umfasst, um eine Stift/Bohrung-Verbindung, vorzugsweise eine Verschraubung zu bilden.

4. Konuspenetrometer nach Anspruch 3, wobei das Kopplungsstück (4) außerdem eine Kopplungsbuchse (42) umfasst, die fest mit dem Kopplungsstift (41) für den Einsatz des vorderen Endes des Hauptkörpers (2) verbunden ist.

5. Konuspenetrometer nach Anspruch 4, wobei die Kopplungsbuchse (42) mit einem Innengewinde (45) versehen ist und das Ende des Hauptkörpers (2) mit einem Außengewinde (42) versehen ist, um eine Verschraubung zu bilden.

6. Konuspenetrometer nach einem der vorhergehenden Ansprüche, wobei sich das vordere Ende der Reibhülse (5) zwischen das vordere Ende des Hauptkörpers (2) erstreckt.

7. Konuspenetrometer nach einem der vorhergehenden Ansprüche, wobei der Hauptkörper (2) aus einem einzigen Stück hergestellt ist.

8. Konuspenetrometer nach einem der vorhergehenden Ansprüche, wobei ein Außengewinde (25) an dem hinteren Ende des Hauptkörpers (2) angeordnet ist, um eine Verbindung mit einem Ende eines Sondierstabes zu bilden.

9. Konuspenetrometer nach einem der vorhergehenden Ansprüche, wobei Kraftmessmittel in dem Hauptkörper (2) angeordnet sind, um den Konuswiderstand zu messen.

10. Konuspenetrometer nach einem der vorhergehenden Ansprüche, wobei das Kraftmesselement (6) als ein hohler Zylinder mit einer im Wesentlichen glatten Innenseite ausgebildet ist.

11. Konuspenetrometer nach einem der vorhergehenden Ansprüche, wobei das Kraftmesselement (6) als ein hohler Zylinder mit einer im Wesentlichen glatten Außenseite ausgebildet ist.

12. Verfahren zur Herstellung eines Konuspenetrometers, umfassend einen im Wesentlichen rohrförmigen Hauptkörper (2) mit einem vorderen Ende und einem hinteren Ende, der einen Absatz (23) mit einer Anschlagfläche (22) aufweist, die zu dem vorderen Ende ausgerichtet ist,
wobei ein im Wesentlichen hülsenförmiges Kraftmesselement (6), das zwei Endabschnitte (64, 65) mit Endflächen (61, 62) aufweist sowie einen mittleren Abschnitt (63), der sich dazwischen erstreckt, rund um einen Teil des Hauptkörpers (2) konzentrisch zu dem Hauptkörper (2) angeordnet ist,
wobei eine zylindrische Reibhülse (5), die einen Absatz (51) mit einer Anschlagfläche (2), welche zu dem hinteren Ende ausgerichtet ist, um einen Abschnitt des Hauptkörpers (2) und um das Kraftmesselement (6) herum angeordnet ist, so dass die Anschlagfläche (52) ihres Absatzes (51), die nach hinten gerichtet ist, an dem Endabschnitt (64) des Kraftmesselements (6) anliegt, und
wobei das Kraftmesselement (6) von dem vorderen Ende in der axialen Richtung über den Hauptkörper (2) geschoben wird, bis es mit der Anschlagfläche (22) des Absatzes (23) in Kontakt kommt, die zu dem vorderen Ende ausgerichtet und auf dem Hauptkörper (2) angeordnet ist,
**dadurch gekennzeichnet, dass**
zunächst das Kraftmesselement (6) über den Hauptkörper (2) geschoben wird, bis es dessen Anschlagfläche (22) erreicht, und anschließend die Reibhülse (5) über den Hauptkörper (2) und das Kraftmesselement (6) von der Vorderseite geschoben wird, wonach eine Nasenbaugruppe, die eine konische Nase (3) umfasst, an das vordere Ende des Hauptkörpers (2) gekoppelt wird,
wobei die Anschlagflächen (22, 52) des Hauptkörpers (2) der jeweiligen Reibhülsen (5) an den Endflächen (61, 62) des Kraftmesselements (6) anliegen, so dass das Kraftmesselement (6) und die Reibhülse (5) axial an dem Hauptkörper (2) formschlüssig fixiert sind, ohne Schraubverbindungen zwischen dem Kraftmesselement (6) und der Reibhülse (5) und zwischen dem Kraftmesselement (6) und dem Hauptkörper (2).

## Revendications

1. Pénétromètre conique comprenant :
- un corps principal (2) sensiblement tubulaire ayant une extrémité avant et une extrémité arrière,
- un nez conique (3) qui est couplé à l'extrémité avant du corps principal (2), et
- un manchon de friction (5) cylindrique disposé autour d'une partie du corps principal (2), de telle manière que le corps principal (2) et le manchon de friction (5) délimitent un dégagement annulaire (10),
dans lequel un élément de mesure de force (6) sensiblement en forme de manchon est logé de manière concentrique avec le corps principal (2) et le manchon de friction (5),
dans lequel le corps principal (2) comprend un épaulement (23) avec une face d'arrêt (22) orientée vers l'extrémité avant,
dans lequel le manchon de friction (5) comprend un épaulement (51) avec une face d'arrêt (52) orientée vers l'extrémité arrière,
dans lequel l'élément de mesure de force (6) a deux parties d'extrémité (64, 65) et une partie centrale (63) s'étendant entre elles, et
dans lequel les parties d'extrémité (64, 65) de l'élément de mesure de force (6) ont des faces d'extrémité (61, 62),
**caractérisé en ce que**
l'élément de mesure de force (6) n'est maintenu dans la direction axiale que par la face d'arrêt (52) orientée vers l'arrière et disposée sur le manchon de friction (5) et par la face d'arrêt (22) orientée vers l'avant et disposée sur le corps principal (2),
dans lequel les faces d'arrêt (22, 52) du corps principal (2), respectivement du manchon de friction (5), s'appliquent contre les faces d'extrémité (61, 62) de l'élément de mesure de force (6), de telle manière que l'élément de mesure de force (6) est fixé sans connexions à vis entre l'élément de mesure de force (6) et le manchon de friction (5) et entre l'élément de mesure de force (6) et le corps principal (2).

2. Pénétromètre conique selon la revendication 1, dans lequel le nez conique (3) est couplé au corps principal (2) au moyen d'une pièce de couplage (4) séparée.

3. Pénétromètre conique selon la revendication 2, dans lequel la pièce de couplage (4) comprend une broche de couplage (41), et le nez conique (3) comprend un trou (31) pour former une connexion broche/trou, de préférence à vis.

4. Pénétromètre conique selon la revendication 3, dans lequel la pièce de couplage (4) comprend également une douille de couplage (42), connectée de manière fixe à la broche de couplage (41), pour l'insertion de l'extrémité avant du corps principal (2).

5. Pénétromètre conique selon la revendication 4, dans lequel la douille de couplage (42) est pourvue d'un filetage interne (45) et l'extrémité du corps principal (2) est pourvue d'un filetage externe (24) afin de former un vissage.

6. Pénétromètre conique selon l'une des revendications précédentes, dans lequel l'extrémité avant du manchon de friction (5) s'étend au-delà de l'extrémité avant du corps principal (2).

7. Pénétromètre conique selon l'une des revendications précédentes, dans lequel le corps principal (2) est réalisé en une seule pièce.

8. Pénétromètre conique selon l'une des revendications précédentes, dans lequel un filetage extérieur (25) est disposé à l'extrémité arrière du corps principal (2) pour former une connexion avec une extrémité d'une tige de sondage.

9. Pénétromètre conique selon l'une des revendications précédentes, dans lequel des moyens de mesure de force sont disposés dans le corps principal (2) pour mesurer la résistance du cône.

10. Pénétromètre conique selon l'une des revendications précédentes, dans lequel l'élément de mesure de force (6) est réalisé sous la forme d'un cylindre creux avec un côté intérieur sensiblement lisse.

11. Pénétromètre conique selon l'une des revendications précédentes, dans lequel l'élément de mesure de force (6) est réalisé sous la forme d'un cylindre creux avec un côté extérieur sensiblement lisse.

12. Procédé de fabrication d'un pénétromètre conique comprenant un corps principal (2) sensiblement tubulaire présentant une extrémité avant et une extrémité arrière, ayant un épaulement (23) avec une face d'arrêt (22) orientée vers l'extrémité avant,
dans lequel un élément de mesure de force (6) sensiblement en forme de manchon, qui a deux parties d'extrémité (64, 65) avec des faces d'extrémité (61, 62) et une partie centrale (63) s'étendant entre elles, est disposé autour d'une partie du corps principal (2), concentriquement au corps principal (2),
dans lequel un manchon de friction (5) cylindrique, qui présente un épaulement (51) avec une face d'arrêt (52) orientée vers l'extrémité arrière, est disposé autour d'une partie du corps principal (2) et autour de l'élément de mesure de force (6), de sorte que la face d'arrêt (52) de l'épaulement (51) de celui-ci qui est orientée vers l'arrière s'applique contre la partie d'extrémité (64) de l'élément de mesure de force (6), et
dans lequel l'élément de mesure de force (6) est déplacé sur le corps principal (2) dans la direction axiale à partir de l'extrémité avant jusqu'à ce qu'il touche la face d'arrêt (22) de l'épaulement (23) qui est dirigé vers l'avant et disposé sur le corps principal (2),
**caractérisé en ce que**
tout d'abord, l'élément de mesure de force (6) est poussé sur le corps principal (2) jusqu'à ce qu'il atteigne la face d'arrêt (22) de celui-ci, et
ensuite le manchon de friction (5) est poussé sur le corps principal (2) et l'élément de mesure de force (6) depuis le côté avant,
après quoi un ensemble de nez comprenant un nez conique (3) est couplé à l'avant du corps principal (2),
dans lequel les faces d'arrêt (22, 52) du corps principal (2) respectivement du manchon de friction (5) s'appliquent sur les faces d'extrémité (61, 62) de l'élément de mesure de force (6), de sorte que l'élément de mesure de force (6) et le manchon de friction (5) sont fixés axialement sur le corps principal (2) de manière à ce qu'ils se verrouillent positivement, sans connexions à vis entre l'élément de mesure de force (6) et le manchon de friction (5) et entre l'élément de mesure de force (6) et le corps principal (2).
